(19) Europäisches Patentamt
European Patent Office
Office européen des brevets

(11) Veröffentlichungsnummer: **0 517 059 A1**

# (12) EUROPÄISCHE PATENTANMELDUNG

(21) Anmeldenummer: **92108757.3**

(51) Int. Cl.5: **A61B 17/58**

(22) Anmeldetag: **25.05.92**

(30) Priorität: **05.06.91 CH 1676/91**

(43) Veröffentlichungstag der Anmeldung: **09.12.92 Patentblatt 92/50**

(84) Benannte Vertragsstaaten: **CH DE FR GB LI**

(71) Anmelder: **Synthes AG Chur**
**Grabenstrasse 15**
**CH-7002 Chur(CH)**

(72) Erfinder: **Schläpfer, Johannes Fridolin, Dr.**
**Leimen**
**W-8750 Glarus(CH)**
Erfinder: **Aebi, Max, Dr.**
**Gartenstrasse 2**
**W-4600 Olten(CH)**

(74) Vertreter: **Lusuardi, Werther Giovanni, Dr.**
**Dr. Lusuardi AG, Kreuzbühlstrasse 8**
**CH-8008 Zürich(CH)**

## (54) Pedikelhaken.

(57) Dieser Pedikelhaken für die Behandlung von Wirbelsäulendeformitäten besteht aus einem an einen Längsträger (1) befestigbaren Schaftteil (2) und einer daran anschliessenden, gebogenen, zweischenkligen Hakenklinge (3), deren lateraler (14) Schenkel länger ist als der mediale (15) Schenkel. Dank dieser asymmetrischen Ausführung der Hakenklinge (3) wird eine Erhöhung der lateralen Begrenzung der Einbuchtung (22) zwischen den beiden Schenkeln (14,15) erreicht. Die Gefahr, dass der Pedikelhaken unter Belastung medial abrutschen und das Rückenmark verletzen kann wird dadurch wesentlich reduziert.

11
2
1
10
7
8
4
5
6
3
15
22
14
9

Fig. 1

EP 0 517 059 A1

Die Erfindung bezieht sich auf einen Pedikelhaken, gemäss dem Oberbegriff des Patentanspruchs 1.

Es sind bereits Pedikelhaken der eingangs genannten Gattung bekannt, beispielsweise aus J.Dubousset und Y.Cotrel in Orthopäde (1989)-18:118-127 "Die CD-Instrumentation in der Behandlung von Wirbelsäulendeformitäten", deren Klinge eine symmetrische Einbuchtung aufweist und wahllos sowohl für den linken als auch den rechten Pedikel des Wirbelkörpers Verwendung finden. Der Zweck der Einbuchtung bei diesem bekannten Pedikelhaken liegt in der Verhinderung des Abrutschens des Pedikelhakens bei seitlicher Belastung, was eine Verletzung der Dura verursachen kann. Demzufolge wird eine möglichst tiefe anatomisch gerechte Einbuchtung angestrebt. Die Einbuchtung der Klinge dieses bekannten Pedikelhakens ist zentrisch angeordnet und völlig symmetrisch. Damit ist deren mediale und laterale Begrenzung gleich hoch. Die Höhe der beiden Schenkel ist durch die Breite des Pedikelhakens und die Form der Einbuchtung limitiert, welche wiederum den anatomischen Gegebenheiten angepasst sind. Nachteilig bei dieser symmetrischen Gestaltung des Pedikelhnkens ist die nach wie vor bestehende Gefahr, dass der Pedikelhaken unter Belastung medial abrutschen und dabei das Rückenmark verletzen kann.

Hier will die Erfindung Abhilfe schaffen. Der Erfindung liegt die Aufgabe zugrunde, einen, eine Links- und eine Rechtsausführung umfassenden Pedikelhaken mit einer asymmetrischen Einbuchtung zu schaffen, der eine erhöhte intraoperative Sicherheit aufweist.

Die Erfindung löst die gestellte Aufgabe mit einem Pedikelhaken, welcher die Merkmale des Anspruchs 1 aufweist.

Die durch die Erfindung erreichten Vorteile sind im wesentlichen darin zu sehen, dass dank der asymmetrischen Ausführung der Einbuchtung eine Erhöhung der lateralen Begrenzung der Einbuchtung erreicht werden kann. Damit wird die Gefahr, dass der Pedikelhaken unter Belastung medial abrutschen und das Rückenmark verletzen kann wesentlich kleiner. Auf der anderen Seite ist die mediale Begrenzung weniger hoch. Dieser Effekt wirkt sich aber nicht negativ aus, solange links und rechts Pedikelhaken montiert werden und die mit den Pedikelhaken verbundenen Längsträger mit Hilfe von Querstäben zu einem geschlossenen Rahmen ergänzt werden, wie dies üblich ist.

Ein weiterer Vorteil der Erfindung besteht darin, dass dank der verlängerten lateralen Begrenzung, der Pedikelhaken intraoperativ schon sehr früh lateral geführt wird, was die Montage erleichtert und sicherer macht.

Die Befestigung des erfindungsgemässen Pedikelhakens an einen Längsträger - innerhalb einer Wirbelsäulenfixationsvorrichtung - kann auf verschiedene, bekannte Arten erfolgen. Zu diesem Zweck weist der Schaftteil des erfindungsgemässen Pedikelhakens entsprechende Konstruktionselemente auf, welche die Verbindung zum Längsträger gestattet, beispielsweise wie in der EP-A 0 348 272 offenbart.

Die klinische Applikation des erfindungsgemässen Pedikelhakens ist völlig analog zu derjenigen bekannter Systeme und ist im Detail in J.Dubousset und Y.Cotrel, Orthopäde (1989)-18:118-127 "Die CD-Instrumentation in der Behandlung von Wirbelsäulendeformitäten" beschrieben.

Ein Ausführungsbeispiel der Erfindung, welches zugleich das Funktionsprinzip erläutert, ist in der Zeichnung dargestellt und wird im folgenden näher beschrieben.

Fig. 1 stellt einen partiellen Axialschnitt durch den erfindungsgemässen Pedikelhaken dar;

Fig. 2 stellt eine Aufsicht auf zwei links und rechts eines Wirbels montierte Pedikelhaken gemäss Fig. 1 dar;

Fig. 3 stellt eine seitliche Ansicht eines erfindungsgemässen, rechts eines Wirbels montierten Pedikelhakens dar;

Fig. 4 stellt eine perspektivische Ansicht eines rechten Pedikelhakens dar;

Fig. 5 stellt einen teilweisen Querschnitt im Bereich der Einbuchtung eines links eines Wirbels montierten Pedikelhakens dar.

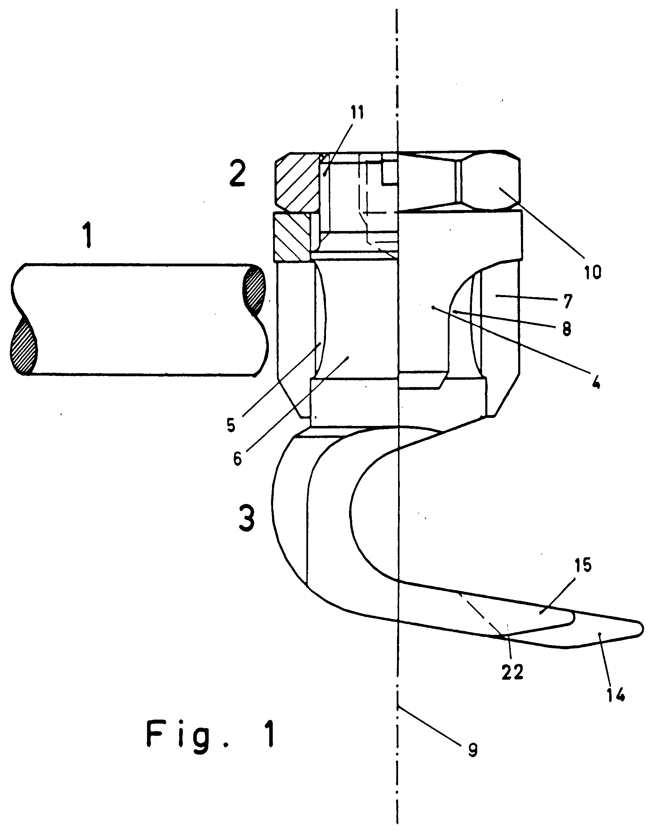

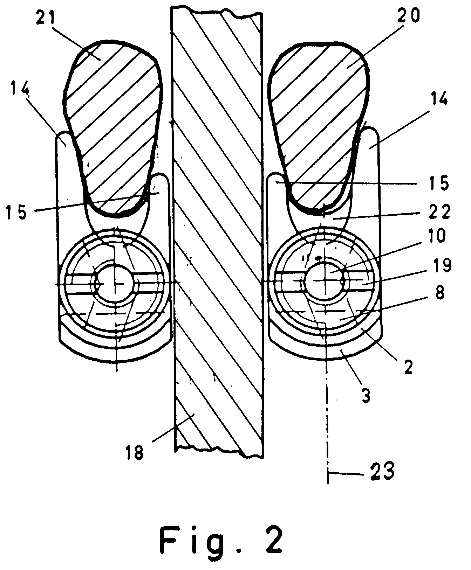

Der in Fig.1 im Detail gezeigte Pedikelhaken besteht im wesentlichen aus einem an einen Längsträger 1 befestigbaren Schafteil 2 und einer daran anschliessenden, gebogenen, an ihrem Ende zweischenkligen Hakenklinge 3. Die beiden Schenkel 14 und 15 der Hakenklinge 3 weisen eine unterschiedliche Länge auf und zwar ist bei der in Fig. 1 dargestellten Linksausführung (für den linken Pedikel 21 des Wirbels) der laterale Schenkel 14 um 3,5 mm länger als der mediale Schenkel 15. Bei der in Fig. 2 ebenfalls gezeigten Rechtsausführung (für den rechts vom Rückenmark 18 gelegenen rechten Pedikel 20 des Wirbels) sind die Verhältnisse genau umgekehrt.

Die Einbuchtung 22 ist im wesentlichen V-förmig gestaltet mit einer lateralen Innenkante 27 und einer medialen Innenkante 28, wobei die beiden Innenkanten 27 und 28 Winkel 25,26 von 10° zur longitudinalen Mittelebene 23 des Pedikelhakens einschliessen. An ihrem Grund 24 ist die Einbuchtung 22 abgerundet.

Bei der bevorzugten Ausführungsform der Erfindung gemäss Fig. 2 ist die zwischen den beiden Schenkeln 14 und 15 liegende Einbuchtung 22 - gegenüber der longitudinalen Mittelebene 23 des Pedikelhakens - zudem um 0,2 mm nach medial verschoben, was automatisch zu ungleich langen Schenkein 14 und 15 führt.

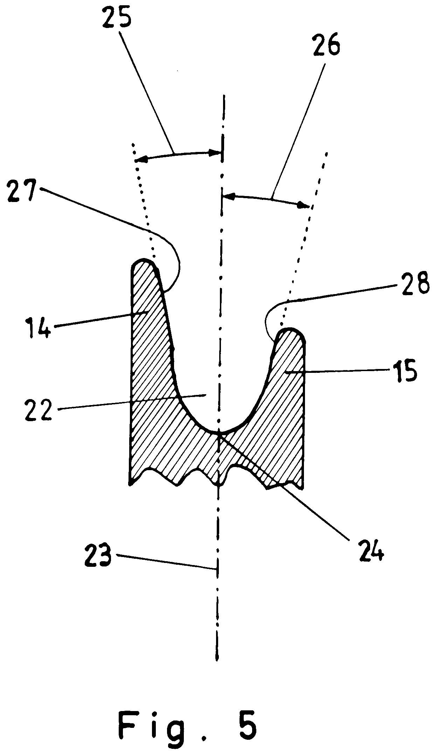

Ungleich lange Schenkel 14 und 15 können aber auch - wie in Fig. 5 dargestellt - durch Wahl unterschiedlicher Winkel 25 und 26 zwischen den lateralen 27 und medialen 28 Innenkanten und der longitudinalen Mittelebene 23 des Pedikelhakens (medial grösser als lateral) oder durch eine Kombination von ungleichen Winkeln 25 und 26 und versetzter Lage der Einbuchtung 22 erhalten werden.

Der Schaftteil 2 besteht bei der in Fig. 1 und 2 gezeigten Ausführungsform aus einer Verbindungseinrichtung, welche in der hängigen Schweizer Patentanmeldung Nr. 1124/91 der gleichen Anmelderin beschrieben ist. Sie besteht im wesentlichen aus einem äusseren, eine kreiszylindrische Bohrung 5 aufweisenden Element 4 und einem inneren, in der Bohrung 5 koaxial verschiebbaren und um seine Achse 9 rotierbaren zylindrischen Element 6. Beide Elemente 4 und 6, besitzen zwei gemeinsame quer zur Achsrichtung 9 der beiden Elemente 4 und 6 gebildete Durchgangsöffnungen 7 und 8 zur Aufnahme des Längstragers 1. Wird der Längstrager 1 in die beiden Bohrungen 7 und 8 eingeführt, so wird die axiale Verschiebbarkeit des inneren Elementes 6 in der Bohrung 5 des äusseren Elementes 4 in einer Richtung blockiert und seine Rotation auf den Winkelbereich der Durchgangsöffnungen 7 und 8 der beiden Elemente 4 und 6 eingeschränkt. Das innere Element 6 ist wegen seiner Durchgangsöffnung 8 gegenüber seiner Achse 9 elastisch deformierbar und weist im weiteren ein Aussengewinde 11 auf, welches mit der Mutter 10 korrespondiert. Bei einer axialen Verspannung des inneren Elementes 6 mittels der Mutter 10, welche eine axiale Verschiebung des inneren Elementes 6 relativ zum äusseren Element 4 bewirkt, erfolgt deshalb eine Aufweitung des elastisch deformierbaren, inneren Elementes 6 und ein Verspannung desselben innerhalb der Bohrung 5 des äusseren Elementes 4 bei gleichzeitiger Blockierung der gesamten Verbindungseinrichtung und des darin eingeführten Längsträgers 1. Das Anziehen, bzw. Lösen der Mutter 10 geschieht mittels zweier zeichnerisch nicht dargestellter Instrumente, eines Steckschlüssels und eines geeigneten Instrumentes, das beim Manipulieren der Mutter 10 den Pedikelhaken in Position hält. Dieses Instrument wird wie ein Schraubenzieher in den Längsschlitz 19 (Fig. 2) der Mutter 10 eingeführt.

Statt der oben beschriebenen bevorzugten Verbindungseinrichtung für den Schaftteil 2 kann auch jedes andere bekannte Verbindungssystem verwendet werden um den Schaftteil 2 an einen Langsträger 1 zu fixieren, beispielsweise gemäss der EP-A1 348 272.

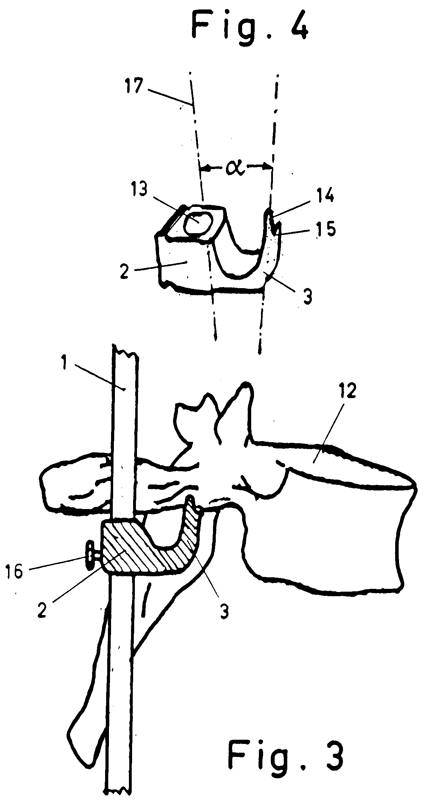

In Fig. 3 ist die Insertion des Pedikelhakens am hinteren Übergang des rechten Pedikels zur Lamina eines Wirbelkörpers 12 dargestellt. Bei der Ausführungsform gemäss Fig. 3 weist der Schaft 2, wie in Fig. 4 im Detail dargestellt, einen Kanal 13 zur Aufnahme des Längsträgers 1 auf, welcher mittels der Schraube 16 fixiert werden kann. Die beiden Schenkel 14 und 15 der Hakenklinge 3 des Pedikelhakens sind gegenüber der durch den Längsträger 1 definierten Längsachse 17 um einen Winkel $\alpha$ von 10° gegen ventral abgebogen.

**Patentansprüche**

1. Pedikelhaken für die Behandlung von Wirbelsäulendeformitäten, mit einem an einen Längsträger (1) befestigbaren Schaftteil (2) und einer daran anschliessenden, gebogenen, eine Einbuchtung (22) aufweisenden Hakenklinge (3), welche durch die Einbuchtung (22) in einen lateralen Schenkel (14) und einen medialen Schenkel (15) aufgeteilt wird, dadurch gekennzeichnet, dass der laterale Schenkel (14) länger als der mediale Schenkel (15) ist.

2. Pedikelhaken nach Anspruch 1, dadurch gekennzeichnet, dass die Einbuchtung (22) V-förmig mit einer lateralen Innenkante (27) und einer medialen Innenkante (28) ausgebildet ist und die beiden Innenkanten (27,28) vorzugsweise einen Winkel von 9 -11° zur longitudinalen Mittelebene (23) des Pedikelhakens einschliessen.

3. Pedikelhaken nach Anspruch 1 oder 2, dadurch gekennzeichnet, dass die Einbuchtung (22) an ihrem Grund (24) abgerundet ist.

4. Pedikelhaken nach einem der Ansprüche 1 - 3, dadurch gekennzeichnet, dass die Längendifferenz zwischen lateralem (14) und medialem (15) Schenkel im Bereich zwischen 2,0 und 5,0 mm, vorzugsweise zwischen 3,0 und 4,0 mm liegt.

5. Pedikelhaken nach einem der Ansprüche 1 - 4, dadurch gekennzeichnet, dass die zwischen dem lateralen (14) und medialen (15) Schenkel liegende Einbuchtung (22) gegenüber der longitudinalen Mittelebene (23) des Pedikelhakens nach medial verschoben ist, vorzugsweise um einen Betrag zwischen 0,1 und 0,3 mm.

6. Pedikelhaken nach einem der Ansprüche 2 - 5 dadurch gekennzeichnet, dass die laterale Innenkante (27) der V-förmigen Einbuchtung (22) einen kleineren Winkel (25) zur longitudinalen Mittelebene (23) des Pedikelhakens einschliesst als die mediale Innenkante (28), wobei die Differenz der beiden Winkel (25,26) vorzugsweise 1 - 2° beträgt.

11
2
1
10
7
8
4
5
6
3
15
22
14
9

Fig. 1

21
20
14
14
15
15
22
10
19
8
2
3
18
23

Fig. 2

Fig. 4
17
α
13
14
15
2
3
1
12
16
2
3
Fig. 3

25
26
27
28
14
15
22
23
24

Fig. 5

Europäisches Patentamt

# EUROPÄISCHER RECHERCHENBERICHT

Nummer der Anmeldung

EP 92 10 8757

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X<br>Y | FR-A-2 627 690 (S.A.LEPINE)<br>* Seite 2, Zeile 40 - Zeile 47; Abbildungen 3-4 * | 1,3-5<br>2 | A61B17/58 |
| Y | FR-A-2 640 493 (BRISTOL-MYERS SQUIBB)<br>* Seite 33, Zeile 32 - Zeile 35; Abbildung 13 * | 2 | |
| A | DE-U-9 006 646 (HOWMEDICA)<br>* Seite 9, Zeile 1 - Seite 10, Zeile 2; Abbildungen 1-4,6 * | 1,4 | |
| D,A | ORTHOPAEDE<br>Nr. 18, 1989,<br>Seiten 118 - 127;<br>J.DUBOUSSET AND Y.COTREL: 'Die CD-Instrumentation in der Behandlung von Wirbelsäulendeformitäten'<br>* Seite 122 'Wirbelklammern' ; Abbildungen 7a-7c * | 1 | |
| | | | RECHERCHIERTE SACHGEBIETE (Int. Cl.5 ) |
| | | | A61B |

Der vorliegende Recherchenbericht wurde für alle Patentansprüche erstellt

| Recherchenort | Abschlußdatum der Recherche | Prüfer |
|---|---|---|
| DEN HAAG | 03 AUGUST 1992 | NICE P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)